# EUROPEAN PATENT APPLICATION

(11) **EP 3 783 015 A1**
(43) Date of publication of application: **24.02.2021**
(21) Application number: 19205484.9
(22) Date of filing: 25.10.2019
(51) Int. Cl.: C07K 14/71, A61P 35/00, C07K 16/32, G01N 33/574, C07K 16/18, A61K 39/00

(54) **MOUSE ANTI-HUMAN ERBB2 MONOCLONAL ANTIBODY 10A6, ENCODING GENE, PREPARATION AND APPLICATION THEREOF**

(30) Priority: 23.08.2019 CN 201910784229
(71) Applicant: Beijing DCTY Biotech Co., Ltd., Beijing 102200 (CN); Jiao, Shunchang, Beijing 102200 (CN)
(72) Inventor: JIAO, Shunchang, Beijing 102200 (CN); ZHANG, Rong, Beijing 102200 (CN); YUAN, Han, Beijing 102200 (CN)
(74) Representative: EP&C

(57) **Abstract**

The present invention provides a mouse anti-human ErbB2 monoclonal antibody 10A6, a preparation method and application thereof, and relates to the technical field of immunobiological cancer therapy, where the heavy-chain amino acid sequence of the mouse anti-human ErbB2 monoclonal antibody 10A6 is depicted in SEQ ID NO.1, and the light chain amino acid sequence thereof is depicted in SEQ ID NO.2. The preparation method of the mouse anti-human ErbB2 monoclonal antibody 10A6 includes the following steps of: 1) immunizing mice with ErbB2-Domain4-encoded protein; 2) fusing the immune spleen cells with myeloma cells; and 3) culturing hybridoma cells, collecting cell culture supernatant, and purifying to acquire the mouse anti-human ErbB2 monoclonal antibody 10A6. In the present invention, the mouse anti-human ErbB2 monoclonal antibody 10A6 features high titer and good specificity and can be used to prepare drugs for diagnosing or treating ErbB2-overexpressed malignancies.

SEQUENCE LISTING

<110>  Beijing DCTY Biotech Co., Ltd.
       JIAO, Shunchang

<120>  MOUSE ANTI-HUMAN ERBB2 MONOCLONAL ANTIBODY 10A6, ENCODING GENE,
       PREPARATION AND APPLICATION THEREOF

<130>  GWP201908328

<150>  201910784229.1
<151>  2019-08-23

<160>  6

<170>  PatentIn version 3.5

<210>  1
<211>  446
<212>  PRT
<213>  mouse

<400>  1

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Ser Thr Ser
            20                  25                  30

Gly Met Gly Val Ser Trp Ile Arg Gln Pro Ser Gly Lys Gly Leu Glu
        35                  40                  45

Trp Leu Ala His Ile Tyr Trp Asp Asp Asp Lys Arg Tyr Asn Pro Ser
    50                  55                  60

Leu Lys Ser Arg Leu Thr Ile Ser Lys Asp Ser Ser Asn Lys Gln Val
65                  70                  75                  80

Phe Leu Lys Phe Thr Ser Val Asp Thr Ala Asp Thr Ala Thr Tyr Tyr
                85                  90                  95

Cys Ala His Phe Ala Tyr Tyr Asn Tyr Asp Gly Ala Val Asp Ser Trp
            100                 105                 110

Gly Gln Gly Thr Ser Val Thr Val Ser Ser Ala Lys Thr Thr Pro Pro
        115                 120                 125

Ser Val Tyr Pro Leu Ala Pro Gly Ser Ala Ala Gln Thr Asn Ser Met
    130                 135                 140

Val Thr Leu Gly Cys Leu Val Lys Gly Tyr Phe Pro Glu Pro Val Thr
145                 150                 155                 160

Val Thr Trp Asn Ser Gly Ser Leu Ser Ser Gly Val His Thr Phe Pro
                165                 170                 175

Ala Val Leu Gln Ser Asp Leu Tyr Thr Leu Ser Ser Ser Val Thr Val
            180                 185                 190

Pro Ser Ser Thr Trp Pro Ser Glu Thr Val Thr Cys Asn Val Ala His
        195                 200                 205

Pro Ala Ser Ser Thr Lys Val Asp Lys Lys Ile Val Pro Arg Asp Cys
    210                 215                 220

Gly Cys Lys Pro Cys Ile Cys Thr Val Pro Glu Val Ser Ser Val Phe
225                 230                 235                 240

Ile Phe Pro Pro Lys Pro Lys Asp Val Leu Thr Ile Thr Leu Thr Pro
                245                 250                 255

Lys Val Thr Cys Val Val Val Asp Ile Ser Lys Asp Asp Pro Glu Val
            260                 265                 270

Gln Phe Ser Trp Phe Val Asp Asp Val Glu Val His Thr Ala Gln Thr
        275                 280                 285

Gln Pro Arg Glu Glu Gln Phe Asn Ser Thr Phe Arg Ser Val Ser Glu
    290                 295                 300

Leu Pro Ile Met His Gln Asp Trp Leu Asn Gly Lys Glu Phe Lys Cys
305                 310                 315                 320

Arg Val Asn Ser Ala Ala Phe Pro Ala Pro Ile Glu Lys Thr Ile Ser
                325                 330                 335

Lys Thr Lys Gly Arg Pro Lys Ala Pro Gln Val Tyr Thr Ile Pro Pro
            340                 345                 350

Pro Lys Glu Gln Met Ala Lys Asp Lys Val Ser Leu Thr Cys Met Ile
        355                 360                 365

Thr Asp Phe Phe Pro Glu Asp Ile Thr Val Glu Trp Gln Trp Asn Gly
    370                 375                 380

Gln Pro Ala Glu Asn Tyr Lys Asn Thr Gln Pro Ile Met Asp Thr Asp
385                 390                 395                 400

Gly Ser Tyr Phe Val Tyr Ser Lys Leu Asn Val Gln Lys Ser Asn Trp
                405                 410                 415

Glu Ala Gly Asn Thr Phe Thr Cys Ser Val Leu His Glu Gly Leu His
        420                 425                 430

Asn His His Thr Glu Lys Ser Leu Ser His Ser Pro Gly Lys
        435                 440                 445

<210>  2
<211>  219
<212>  PRT
<213>  mouse

<400>  2

Asp Ile Val Met Ser Gln Ser Pro Ser Ser Leu Thr Val Ser Ala Gly
1               5                   10                  15

Glu Lys Val Ser Met Ser Cys Lys Ser Ser Gln Ser Leu Leu His Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Lys Gln
                85                  90                  95

Ser Tyr Ser Leu Tyr Thr Phe Gly Gly Gly Thr Lys Leu Glu Ile Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

Pro Ile Val Lys Ser Phe Asn Arg Asn Glu Cys
    210                 215

<210>  3
<211>  1338
<212>  DNA
<213>  mouse

<400>  3
caggttacac tgaaagagtc tggccctggg atattgcagc cctcccagac cctcagtctg      60

acttgctcct tctctggatt ttcattgagc acttctggta tgggtgtgag ctggattcgt     120

cagccttcag gaaagggtct ggagtggctg gcacacattt actgggatga tgacaagcgc     180

tataacccat ccctgaagag ccggctcaca atctccaagg attcctccaa caagcaggtt     240

ttcctcaagt tcaccagtgt tgacactgca gatactgcca catactactg tgctcacttc     300

gcctactata actacgacgg ggctgtggac tcctggggtc aaggaacctc agtcaccgtc     360

tcctcagcca aaacgacacc cccatctgtc tatccactgg cccctggatc tgctgcccaa     420

actaactcca tggtgaccct gggatgcctg gtcaagggct atttccctga gccagtgaca     480

gtgacctgga actctggatc cctgtccagc ggtgtgcaca ccttcccagc tgtcctgcag     540

tctgacctct acactctgag cagctcagtg actgtcccct ccagcacctg gcccagcgag     600

accgtcacct gcaacgttgc ccacccggcc agcagcacca aggtggacaa gaaaattgtg     660

cccagggatt gtggttgtaa gccttgcata tgtacagtcc cagaagtatc atctgtcttc     720

atcttccccc caaagcccaa ggatgtgctc accattactc tgactcctaa ggtcacgtgt     780

gttgtggtag acatcagcaa ggatgatccc gaggtccagt tcagctggtt tgtagatgat     840

gtggaggtgc acacagctca gacgcaaccc cgggaggagc agttcaacag cactttccgc     900

tcagtcagtg aacttcccat catgcaccag gactggctca atggcaagga gttcaaatgc     960

agggtcaaca gtgcagcttt ccctgccccc atcgagaaaa ccatctccaa aaccaaaggc    1020

agaccgaagg ctccacaggt gtacaccatt ccacctccca aggagcagat ggccaaggat    1080

aaagtcagtc tgacctgcat gataacagac ttcttccctg aagacattac tgtggagtgg    1140

cagtggaatg ggcagccagc ggagaactac aagaacactc agcccatcat ggacacagat    1200

ggctcttact tcgtctacag caagctcaat gtgcagaaga gcaactggga ggcaggaaat    1260

actttcacct gctctgtgtt acatgagggc ctgcacaacc accatactga gaagagcctc    1320

tcccactctc ctggtaaa                                                  1338

<210>  4
<211>  657
<212>  DNA
<213>  mouse

<400>  4
gacatagtga tgtcacagtc tccatcctcc ctgactgtgt cagcaggaga gaaggtctct      60

atgagctgca aatccagtca gagtctgctc cacagtagaa cccgaaagaa ctacttggct     120

tggtaccagc agaaaccagg gcagtctcct aaactgctga tctattgggc atccactagg     180

gaatctgggg tccctgatcg cttcacaggc agtggatctg ggacagattt cactctcacc     240

atcagcagtg tgcaggctga agacctggca gtttattact gcaagcaatc ttatagtctg     300

tacacgttcg gaggggggac caagctggaa ataaaacggg ctgatgctgc accaactgta     360

tccatcttcc caccatccag tgagcagtta acatctggag gtgcctcagt cgtgtgcttc     420

ttgaacaact tctaccccaa agacatcaat gtcaagtgga agattgatgg cagtgaacga     480

caaaatggcg tcctgaacag ttggactgat caggacagca aagacagcac ctacagcatg     540

agcagcaccc tcacgttgac caaggacgag tatgaacgac ataacagcta tacctgtgag     600

gccactcaca agacatcaac ttcacccatt gtcaagagct tcaacaggaa tgagtgt        657


<210>  5
<211>  564
<212>  DNA
<213>  artificial sequence

<220>
<223>  ErbB2-Domain4-encoded protein

<400>  5
   atggagctgg cggccttgtg ccgctggggg ctcctcctcg ccctcttgcc ccccggagcc      60
   gcgagcacct gccaccagct gtgcgcccga gggcactgct ggggtccagg gcccacccag     120
   tgtgtcaact gcagccagtt ccttcggggc caggagtgcg tggaggaatg ccgagtactg     180
   caggggctcc ccagggagta tgtgaatgcc aggcactgtt tgccgtgcca ccctgagtgt     240
   cagccccaga atggctcagt gacctgtttt ggaccggagg ctgaccagtg tgtggcctgt     300
   gcccactata aggaccctcc cttctgcgtg gcccgctgcc ccagcggtgt gaaacctgac     360
   ctctcctaca tgcccatctg gaagtttcca gatgaggagg gcgcatgcca gccttgcccc     420
   atcaactgca cccactcctg tgtggacctg gatgacaagg gctgccccgc cgagcagaga     480
   gccagccctc tgacgtccat catctctgcg gtggttggca ttctgctggt cgtggtcttg     540
   ggggtggtct ttgggatcct catc                                             564


<210>  6
<211>  188
<212>  PRT
<213>  artificial sequence

<220>
<223>  the ErbB2-Domain4-encoded protein

<400>  6

Met Glu Leu Ala Ala Leu Cys Arg Trp Gly Leu Leu Leu Ala Leu Leu
1               5                   10                  15

Pro Pro Gly Ala Ala Ser Thr Cys His Gln Leu Cys Ala Arg Gly His
            20                  25                  30

Cys Trp Gly Pro Gly Pro Thr Gln Cys Val Asn Cys Ser Gln Phe Leu
        35                  40                  45

Arg Gly Gln Glu Cys Val Glu Glu Cys Arg Val Leu Gln Gly Leu Pro
    50                  55                  60

Arg Glu Tyr Val Asn Ala Arg His Cys Leu Pro Cys His Pro Glu Cys
65                  70                  75                  80

Gln Pro Gln Asn Gly Ser Val Thr Cys Phe Gly Pro Glu Ala Asp Gln
                85                  90                  95

Cys Val Ala Cys Ala His Tyr Lys Asp Pro Pro Phe Cys Val Ala Arg
            100                 105                 110

Cys Pro Ser Gly Val Lys Pro Asp Leu Ser Tyr Met Pro Ile Trp Lys
        115                 120                 125

Phe Pro Asp Glu Glu Gly Ala Cys Gln Pro Cys Pro Ile Asn Cys Thr
    130                 135                 140

His Ser Cys Val Asp Leu Asp Asp Lys Gly Cys Pro Ala Glu Gln Arg
145                 150                 155                 160

Ala Ser Pro Leu Thr Ser Ile Ile Ser Ala Val Val Gly Ile Leu Leu
                165                 170                 175

Val Val Val Leu Gly Val Val Phe Gly Ile Leu Ile
            180                 185

## Description

### TECHNICAL FIELD

The present invention relates to the technical field of immunobiological cancer therapy, and in particular to a mouse anti-human ErbB2 monoclonal antibody 10A6, an encoding gene, a preparation method and application thereof.

### BACKGROUND ART

ErbB2 (also known as ErbB2, NEU, and CD340) is a proto-oncogene erbB-2-encoded 185 kDa cell membrane receptor, which is one of the members of the epidermal growth factor receptor (EGFR) family.

The activity of Ras-MAPK and PI3K-Ak signaling in ErbB2-overexpressed tumor cells enables strong cell proliferation ability, inhibition of mechanisms of differentiation, maturation and apoptosis, and high malignant degree of cells. ErbB2-overexpressed tumor cells can resist apoptotic effects induced by TNF-α, rays, and a variety of chemotherapeutic drugs.

Clinically, expression of ErbB2 is closely related to patients' prognosis; ErbB2-overexpressed patients are liable to tumor metastases, with short survival time. ErbB2 has been an ideal target for immunobiological cancer therapy and also a focus in the research field of current tumor therapy because there is a significant difference in the expression level of ErbB2 between normal cells and tumor cells.

However, the existing types of targeting ErbB2 monoclonal antibodies are less, with poor efficacy.

### SUMMARY OF THE INVENTION

In view of this, an objective of the present invention is to provide a novel mouse anti-human ErbB2 monoclonal antibody 10A6, a preparation method and application thereof.

To achieve the foregoing invention objective, the present invention provides the following technical solutions:
The present invention provides a mouse anti-human ErbB2 monoclonal antibody 10A6, where a heavy chain amino acid sequence of the mouse anti-human ErbB2 monoclonal antibody 10A6 is depicted in SEQ ID NO.1, and a light chain amino acid sequence thereof is depicted in SEQ ID NO.2.

The present invention provides a gene encoding the mouse anti-human ErbB2 monoclonal antibody 10A6, where a nucleotide sequence encoding the heavy chain of the mouse anti-human ErbB2 monoclonal antibody 10A6 is depicted in SEQ ID NO.3, and a nucleotide sequence encoding the light chain thereof is depicted in SEQ ID NO.4.

The present invention provides a preparation method of the mouse anti-human ErbB2 monoclonal antibody 10A6, including the following steps of:
1) immunizing mice with ErbB2-Domain4-encoded protein as an antigen to acquire immune spleen cells;
2) fusing the immune spleen cells with myeloma cells to acquire hybridoma cells; and
3) culturing the hybridoma cells, collecting cell culture supernatant, and purifying to acquire the mouse anti-human ErbB2 monoclonal antibody 10A6.

Preferably, a nucleotide sequence of the ErbB2-Domain4 gene is depicted in SEQ ID NO.5.

Preferably, an amino acid sequence of the ErbB2-Domain4-encoded protein is depicted in SEQ ID NO.6.

The present invention provides an application of the mouse anti-human ErbB2 monoclonal antibody 10A6 in the preparation of drugs for diagnosing or treating ErbB2-overexpressed malignancies.

The present invention provides an application of the gene encoding the mouse anti-human ErbB2 monoclonal antibody 10A6 in the preparation of drugs for diagnosing or treating ErbB2-overexpressed malignancies.

**Beneficial effects of the present invention:** The present invention provides a mouse anti-human ErbB2 monoclonal antibody 10A6, a gene encoding the mouse anti-human ErbB2 monoclonal antibody 10A6, a preparation method of the mouse anti-human ErbB2 monoclonal antibody 10A6, and application thereof; the mouse anti-human ErbB2 monoclonal antibody 10A6 of the present invention has a high titer, exhibit high affinity for naturally conformational ErbB2 protein, and weakly binds to an ErbB2-negative target cell, with good specificity, being applicable to prepare drugs for diagnosing or treating ErbB2-overexpressed malignancies.

### BRIEF DESCRIPTION OF THE DRAWINGS

FIG. 1 shows a diagram of a binding domain of mouse anti-human ErbB2 monoclonal antibody 10A6;
FIG. 2 shows EC50 values of different monoclonal antibodies screened;
FIG. 3 shows subtype analysis results of the mouse anti-human ErbB2 monoclonal antibody 10A6;
FIG. 4 shows affinities of mouse anti-human ErbB2 monoclonal antibody for target cells analyzed by flow cytometry;
FIG. 5 shows the binding specificity of hybridoma antibody to target cells.

### DETAILED DESCRIPTION

The present invention provides a mouse anti-human ErbB2 monoclonal antibody 10A6, where a heavy-chain amino acid sequence of the mouse anti-human ErbB2 monoclonal antibody 10A6 is depicted in SEQ ID NO.1, and a light chain amino acid sequence thereof is depicted in SEQ ID NO.2.

In the present invention, the heavy chain amino acid sequence of the mouse anti-human ErbB2 monoclonal antibody 10A6 is as follows:
QVTLKESGPGILQPSQTLSLTCSFSGFSLSTS**GMGVS**WIRQPSGKGLEWLA**HIYWDD DKRYNPSLKSRLT**ISKDSSNKQVFLKFTSVDTADTATYYCAH**FAYYNYDGAVDS**WGQG TSVTVSSAKTTPPSVYPLAPGSAAQTNSMVTLGCLVKGYFPEPVTVTWNSGSLSSGVHTF PAVLQSDLYTLSSSVTVPSSTWPSETVTCNVAHPASSTKVDKKIVPRDCGCKPCICTVPEVS SVFIFPPKPKDVLTITLTPKVTCVVVDISKDDPEVQFSWFVDDVEVHTAQTQPREEQFNST FRSVSELPIMHQDWLNGKEFKCRVNSAAFPAPIEKTISKTKGRPKAPQVYTIPPPKEQMAK DKVSLTCMITDFFPEDITVEWQWNGQPAENYKNTQPIMDTDGSYFVYSKLNVQKSNWE AGNTFTCSVLHEGLHNHHTEKSLSHSPGK (SEQ ID No.1) ; the heavy chain of the mouse anti-human ErbB2 monoclonal antibody 10A6 includes a total of 446 amino acids, where the bold amino acids are CDR region, and the underlined part is CH123 domain, a constant region.

In the present invention, the light chain amino acid sequence of the mouse anti-human ErbB2 monoclonal antibody 10A6 is as follows:
DIVMSQSPSSLTVSAGEKVSMSC**KSSQSLLHSRTRKNYLA**WYQQKPGQSPKLLIY**W ASTRES**GVPDRFTGSGSGTDFTLTISSVQAEDLAVYYC**KQSYSLYT**FGGGTKLEIKRADA APTVSIFPPSSEQLTSGGASVVCFLNNFYPKDINVKWKIDGSERQNGVLNSWTDQDSKDS TYSMSSTLTLTKDEYERHNSYTCEATHKTSTSPIVKSFNRNEC (SEQ ID No.2); the light chain of the mouse anti-human ErbB2 monoclonal antibody 10A6 includes a total of 219 amino acids, where the bold amino acids are CDR region, and the underlined part is CH123 domain, a constant region.

The present invention provides a gene encoding the mouse anti-human ErbB2 monoclonal antibody 10A6. A nucleotide sequence encoding the heavy chain of the mouse anti-human ErbB2 monoclonal antibody 10A6 is depicted in SEQ ID NO.3, specifically as follows:
CAGGTTACACTGAAAGAGTCTGGCCCTGGGATATTGCAGCCCTCCCAGACCCTCA GTCTGACTTGCTCCTTCTCTGGATTTTCATTGAGCACTTCTGGTATGGGTGTGAGCTGG ATTCGTCAGCCTTCAGGAAAGGGTCTGGAGTGGCTGGCACACATTTACTGGGATGATG ACAAGCGCTATAACCCATCCCTGAAGAGCCGGCTCACAATCTCCAAGGATTCCTCCAA CAAGCAGGTTTTCCTCAAGTTCACCAGTGTTGACACTGCAGATACTGCCACATACTAC TGTGCTCACTTCGCCTACTATAACTACGACGGGGCTGTGGACTCCTGGGGTCAAGGAA CCTCAGTCACCGTCTCCTCAGCCAAAACGACACCCCCATCTGTCTATCCACTGGCCCC TGGATCTGCTGCCCAAACTAACTCCATGGTGACCCTGGGATGCCTGGTCAAGGGCTAT TTCCCTGAGCCAGTGACAGTGACCTGGAACTCTGGATCCCTGTCCAGCGGTGTGCAC ACCTTCCCAGCTGTCCTGCAGTCTGACCTCTACACTCTGAGCAGCTCAGTGACTGTCC CCTCCAGCACCTGGCCCAGCGAGACCGTCACCTGCAACGTTGCCCACCCGGCCAGCA GCACCAAGGTGGACAAGAAAATTGTGCCCAGGGATTGTGGTTGTAAGCCTTGCATATG TACAGTCCCAGAAGTATCATCTGTCTTCATCTTCCCCCCAAAGCCCAAGGATGTGCTC ACCATTACTCTGACTCCTAAGGTCACGTGTGTTGTGGTAGACATCAGCAAGGATGATC CCGAGGTCCAGTTCAGCTGGTTTGTAGATGATGTGGAGGTGCACACAGCTCAGACGC AACCCCGGGAGGAGCAGTTCAACAGCACTTTCCGCTCAGTCAGTGAACTTCCCATCA TGCACCAGGACTGGCTCAATGGCAAGGAGTTCAAATGCAGGGTCAACAGTGCAGCTT TCCCTGCCCCCATCGAGAAAACCATCTCCAAAACCAAAGGCAGACCGAAGGCTCCAC AGGTGTACACCATTCCACCTCCCAAGGAGCAGATGGCCAAGGATAAAGTCAGTCTGA CCTGCATGATAACAGACTTCTTCCCTGAAGACATTACTGTGGAGTGGCAGTGGAATGG GCAGCCAGCGGAGAACTACAAGAACACTCAGCCCATCATGGACACAGATGGCTCTTA CTTCGTCTACAGCAAGCTCAATGTGCAGAAGAGCAACTGGGAGGCAGGAAATACTTT CACCTGCTCTGTGTTACATGAGGGCCTGCACAACCACCATACTGAGAAGAGCCTCTCC CACTCTCCTGGTAAA. In the present invention, a nucleotide sequence encoding the light chain of the mouse anti-human ErbB2 monoclonal antibody 10A6 is depicted in SEQ ID NO.4, specifically as follows:

GACATAGTGATGTCACAGTCTCCATCCTCCCTGACTGTGTCAGCAGGAGAGAAGG
TCTCTATGAGCTGCAAATCCAGTCAGAGTCTGCTCCACAGTAGAACCCGAAAGAACTA
CTTGGCTTGGTACCAGCAGAAACCAGGGCAGTCTCCTAAACTGCTGATCTATTGGGCA
TCCACTAGGGAATCTGGGGTCCCTGATCGCTTCACAGGCAGTGGATCTGGGACAGATT
TCACTCTCACCATCAGCAGTGTGCAGGCTGAAGACCTGGCAGTTTATTACTGCAAGCA
ATCTTATAGTCTGTACACGTTCGGAGGGGGGACCAAGCTGGAAATAAAACGGGCTGAT
GCTGCACCAACTGTATCCATCTTCCCACCATCCAGTGAGCAGTTAACATCTGGAGGTG
CCTCAGTCGTGTGCTTCTTGAACAACTTCTACCCCAAAGACATCAATGTCAAGTGGAA
GATTGATGGCAGTGAACGACAAAATGGCGTCCTGAACAGTTGGACTGATCAGGACAG
CAAAGACAGCACCTACAGCATGAGCAGCACCCTCACGTTGACCAAGGACGAGTATGA
ACGACATAACAGCTATACCTGTGAGGCCACTCACAAGACATCAACTTCACCCATTGTC
AAGAGCTTCAACAGGAATGAGTGT.

The present invention provides a preparation method of the mouse anti-human ErbB2 monoclonal antibody 10A6, including the following steps of: 1) immunizing mice with ErbB2-Domain4-encoded protein as an antigen to acquire immune spleen cells; 2) fusing the immune spleen cells with myeloma cells to acquire hybridoma cells; and 3) culturing the hybridoma cells, collecting cell culture supernatant, and purifying to acquire the mouse anti-human ErbB2 monoclonal antibody 10A6.

In the present invention, first of all, mice are immunized with ErbB2-Domain4-encoded protein as an antigen to acquire immune spleen cells. In the present invention, the mice are preferably female BALB/c mice aged 6-8 weeks, the frequency of the immunization is preferably three times, and the dose of the first two immunizations is preferably 12.5 µg/mouse; the interval between the first two immunizations is preferably two weeks; the time of the third immunization is preferably at 3 days prior to fusion of immune spleen cells with myeloma cells, and the dose of the third immunization is preferably 60 µg/mouse. In the present invention, immune spleen cells are harvested after the immunization; the harvesting method of the immune spleen cells is preferably to harvest immunized mouse spleens, grind the mouse spleens, and then pass through a cell mesh to acquire the immune spleen cells.

In the present invention, preparation of the ErbB2-Domain4-encoded protein preferably includes the following steps: S1) artificially synthesizing ErbB2-Domain4 gene, and then ligating the ErbB2-Domain4 gene to a vector to acquire a recombinant vector; and S2) transfecting the recombinant vector into *Escherichia coli* (*E. coli*), disrupting the cells, collecting cell disruption supernatant, conducting affinity purification with a nickel column, and collecting the ErbB2-Domain4-encoded protein. In the present invention, a nucleotide sequence of the ErbB2-Domain4 gene is depicted in SEQ ID NO.5, specifically as follows:

ATGGAGCTGGCGGCCTTGTGCCGCTGGGGGCTCCTCCTCGCCCTCTTGCCCCCCG
GAGCCGCGAGCACCTGCCACCAGCTGTGCGCCCGAGGGCACTGCTGGGGTCCAGGG
CCCACCCAGTGTGTCAACTGCAGCCAGTTCCTTCGGGGCCAGGAGTGCGTGGAGGAA
TGCCGAGTACTGCAGGGGCTCCCCAGGGAGTATGTGAATGCCAGGCACTGTTTGCCG
TGCCACCCTGAGTGTCAGCCCCAGAATGGCTCAGTGACCTGTTTTGGACCGGAGGCT
GACCAGTGTGTGGCCTGTGCCCACTATAAGGACCCTCCCTTCTGCGTGGCCCGCTGCC
CCAGCGGTGTGAAACCTGACCTCTCCTACATGCCCATCTGGAAGTTTCCAGATGAGGA
GGGCGCATGCCAGCCTTGCCCCATCAACTGCACCCACTCCTGTGTGGACCTGGATGAC
AAGGGCTGCCCCGCCGAGCAGAGAGCCAGCCCTCTGACGTCCATCATCTCTGCGGTG
GTTGGCATTCTGCTGGTCGTGGTCTTGGGGGTGGTCTTTGGGATCCTCATC.

In the present invention, an amino acid sequence of the ErbB2-Domain4-encoded protein is depicted in SEQ ID NO.6, specifically as follows:

MELAALCRWGLLLALLPPGAASTCHQLCARGHCWGPGPTQCVNCSQFLRGQECVE
ECRVLQGLPREYVNARHCLPCHPECQPQNGSVTCFGPEADQCVACAHYKDPPFCVARCP
SGVKPDLSYMPIWKFPDEEGACQPCPINCTHSCVDLDDKGCPAEQRASPLTSIISAVVGILL
VVVLGVVFGILI.

In the present invention, after ligation of both ends of the ErbB2-Domain4 gene to restriction sites, the ErbB2-Domain4 gene and the vector are separately double digested and ligated; enzymes for the double digestion are preferably restriction endonucleases NdeI and XhoI. In the present invention, the vector is preferably pET-28a plasmid. The detailed steps and parameter setting of the double digestion and ligation are not particularly limited in the present invention, but the detailed steps and parameters of conventional double digestion and ligation in the art may be used. See embodiments for details.

In the present invention, after the acquisition of the recombinant vector, the recombinant vector is transfected into *E. coli* for culture, the cells are disrupted, the cell disruption supernatant is collected, the affinity purification is conducted with a nickel column, and the ErbB2-Domain4-encoded protein is collected. In the present invention, the *E. coli* is preferably *E. coli* BL21(DE3); in the present invention, the method for transfecting the recombinant vector into *E. coli* is preferably a chemical method, by which the recombinant vector is transfected into *E. coli* competent cells using heat shock. In the present invention, after transfecting the recombinant vector into the *E. coli* competent cells, cell culture is conducted, and cell clones are picked up for sequencing and verification; correctly sequenced and verified cell clones are selected to culture; in the process of the culture in the present invention, bacterial cells are harvested after induction with IPTG. In the present invention, the cell disruption method is preferably ultrasonic fragmentation, and the detailed steps are described in embodiments.

In the present invention, after the acquisition of immune spleen cells, the immune spleen cells are fused with myeloma cells to acquire hybridoma cells. In the present invention, the myeloma cells are preferably mouse myeloma SP2/0 cells, and the mouse myeloma SP2/0 cells are purchased from Cell Resource Center, Institute of Basic Medical Sciences (IBMS) of the Chinese Academy of Medical Sciences (CAMS); the mouse myeloma SP2/0 cells are preferably cultured in 10% FBS supplemented DMEM; in the present invention, the mouse myeloma SP2/0 cells are preferably subcultured at a 1:3 ratio every 24 h for use. In the present invention, the preferred steps of the cell fusion are as follows: wash the immune spleen cells and the mouse myeloma SP2/0 cells thrice with DMEM separately, mix the immune spleen cells with the mouse myeloma SP2/0 cells, and culture them after reaction with 50% PEG1500 for 1-2 min. In the present invention, the quantity proportion of the immune spleen cells and the mouse myeloma SP2/0 cells is preferably 1:(8-12), and more preferably 1:10. In the present invention, the medium for the culture is preferably HAT supplemented complete medium, and the culture is preferably conducted in a 96-well plate; the environmental conditions of the culture are preferably at 37°C in a 5% CO₂ atmosphere.

In the present invention, after the cell fusion, positive hybridoma cells are preferably screened and cloned; in the present invention, the screening of the positive hybridoma cells is preferably conducted by means of indirect ELISA, and the positive hybridoma cells are defined as cells in cell wells which are determined as positive by means of indirect ELISA detection continuously; in the present invention, after the acquisition of the positive hybridoma cells, subcloning is preferably conducted by means of limiting dilution; wells of hybridoma cells with high titer, monoclonal growth and good morphology are selected to sequentially reclone in the same manner; the frequency of the recloning is preferably at least two times; in the present invention, the hybridoma cells in the cloned positive wells are cultured till the hybridoma cells grow well, followed by cryopreservation of the hybridoma cells.

In the present invention, after the acquisition of the hybridoma cells, the hybridoma cells are cultured, and the cell culture supernatant is collected and purified to acquire the mouse anti-human ErbB2 monoclonal antibody 10A6. In the present invention, the medium for culturing the hybridoma cells is preferably 10% fetal bovine serum (FBS) supplemented DMEM, and the seeding density of the hybridoma cell culture is preferably 10e6/mL; the passage number and time of the hybridoma cells are not particularly limited in the present invention, but those capable of acquiring a significant number of cells may be used.

In the present invention, after the acquisition of the cells, the cell culture supernatant is collected. In the present invention, the collection method of the cell culture supernatant is preferably centrifugation. The rotational speed and time of the centrifugation are not particularly limited in the present invention, but those capable of achieving cell removal may be used. In the present invention, after the acquisition of the supernatant, the supernatant is purified to acquire the mouse anti-human ErbB2 monoclonal antibody 10A6; in the present invention, the purification is preferably conducted through HiTrap protein G affinity chromatography. The detailed steps and parameter setting of the HiTrap protein G affinity chromatography are not particularly limited in the present invention, but detailed steps and parameter setting of conventional HiTrap protein G affinity chromatography in the art may be used.

The present invention further provides an application of the mouse anti-human ErbB2 monoclonal antibody 10A6 in the preparation of drugs for diagnosing or treating ErbB2-overexpressed malignancies.

The present invention further provides an application of the gene encoding the mouse anti-human ErbB2 monoclonal antibody 10A6 in the preparation of drugs for diagnosing or treating ErbB2-overexpressed malignancies.

The technical solution provided by the present invention will be described in detail in connection with the following embodiments, but they should not be construed as limiting the claimed scope of the present invention.

### Embodiment 1

Mouse anti-human ErbB2 monoclonal antibody 10A6

### S1, synthesis of ErbB2-Domain4 gene

The nucleic acid sequence of ErbB2-Domain4 gene is as follows:

atggagctggcggccttgtgccgctgggggctcctcctcgccctcttgccccccggagccgcgagcacctgccaccagctgtgcgc
ccgagggcactgctggggtccagggcccacccagtgtgtcaactgcagccagttccttcggggccaggagtgcgtggaggaatgccgag
tactgcaggggctccccagggagtatgtgaatgccaggcactgtttgccgtgccaccctgagtgtcagccccagaatggctcagtgacctgt
tttggaccggaggctgaccagtgtgtggcctgtgcccactataaggaccctcccttctgcgtggcccgctgccccagcggtgtgaaacctga
cctctcctacatgcccatctggaagtttccagatgaggagggcgcatgccagccttgccccatcaactgcacccactcctgtgtggacctgga
tgacaagggctgccccgccgagcagagagccagccctctgacgtccatcatctctgcggtggttggcattctgctggtcgtggtcttgggggg
tggtctttgggatcctcatc

During synthesis, restriction sites of restriction endonucleases NdeI and XhoI were separately added to the upstream and downstream of the ErbB2-Domain4 gene to acquire ErbB2-Domain4 insert.

### S2, expression and purification of ErbB2-Domain4 protein

Using 30 IU each of restriction endonucleases NdeI and XhoI (Neb), 20 µg of the vector pET-28a plasmid and the ErbB2-Domain4 gene insert were separately digested for 1 h, 50 ng each of restriction fragments was recovered, and the digested pET-28a plasmid was ligated with 5000 U(Neb) of T4 ligase for 1 h.

The ligated recombinant vector was heat shocked for 90 s and transformed into chemically competent *Escherichia coli* (*E. coli*) BL21(DE3) (purchased from Beijing TransGen Biotech Co., Ltd.). Clones were picked up for sequencing and verification, and correctly verified strains were seeded in LB medium supplemented with 50 µg/ml ampicillin to culture butterfly seed liquid overnight. One milliliter of the seed liquid was seeded into 100 mL of LB medium and cultured for 2-4 h till mid-log phase (spectrophotometrically, absorbance at 550 nm ranged from 5.0 to 1.0). Then, IPTG was added to a final concentration of 1 mmol/L, followed by induction for 2 h and then centrifugation for 15 min at 5000 ×g to collect bacterial cells; after each gram of bacterial cells were resuspended with 3 mL of STE buffer (purchased from Beijing Solarbio Science & Technology Co., Ltd.), each gram of wet *E. coli* cells were agitated with 4 µL of 0.1 mol/L PMSF and 80 µl of 10 mg/ml lysozyme for 20 min, followed by 4 mg of deoxycholic acid per gram of wet *E. coli* cells. The bacterial cells were disrupted by ultrasonic cell disruptor (VCX600, Sonics and Materials, Inc.) for 12 min. The ultrasonic mode was 45% energy at 45 s interval every 15 working days.

After centrifugation, precipitates were collected. Inclusion bodies were oscillated and washed with STE buffer containing 0.5% Triton X100 for 15 min, and then were collected by centrifugation at 15000 ×g. The inclusion bodies were dissolved in STE buffer supplemented with 8 M urea and 0.1 mol/L PMSF, and then pH value was adjusted to 8.0 with hydrochloric acid. Protein was purified through Ni column affinity chromatography; ErbB2-Domain4 protein was collected as antigen for use. The amino acid sequence of the ErbB2-Domain4 protein is as follows:

MELAALCRWGLLLALLPPGAASTCHQLCARGHCWGPGPTQCVNCSQFLRGQECVE
ECRVLQGLPREYVNARHCLPCHPECQPQNGSVTCFGPEADQCVACAHYKDPPFCVARCP
SGVKPDLSYMPIWKFPDEEGACQPCPINCTHSCVDLDDKGCPAEQRASPLTSIISAVVGILL
VVVLGVVFGILI

### S3, immunization of mice:

Using the purified ErbB2-Domain4 protein as antigen, three female BALB/c mice aged 6-8 weeks were immunized thrice. The interval between the first two immunizations was two weeks; the dose was 12.5 µg/mouse; tail blood was sampled at 10 days after the second immunization. The last recombinant antigen injection was given via the tail vein at 3 days before fusion; the dose was 60 µg/mouse; mice were boosted once.

### S4, fusion of myeloma cells with immune spleen cells:

Mouse myeloma SP2/0 cells (purchased from Cell Resource Center, Institute of Basic Medical Sciences of the Chinese Academy of Medical Sciences) were cultured in 10% FBS supplemented DMEM and subcultured at a 1:3 vaccination ratio every 24 h for use. Immune mouse spleens were collected, ground, and passed through a cell mesh to acquire mouse spleen cells. After mouse spleen cells and SP2/0 cells were washed with DMEM thrice, the ratio of spleen cell to SP2/0 cell was adjusted to 1:10; after reaction with 50% PEG1500 for 1-2 min, cells were diluted with HAT supplemented complete medium, seeded in a 96-well plate, and cultured at 37°C in a 5% CO₂ atmosphere.

### S5, screening and cloning of positive hybridoma cells:

Cell wells which were determined as positive by means of indirect ELISA in duplicate consecutively were subcloned by means of limiting dilution. In case of specific anti-ErbB2 antibodies determined in cell wells, wells of hybridoma cells with high antibody titer, monoclonal growth and good morphology were selected to sequentially reclone in the same manner, at least two times; the hybridoma cells in the cloned positive wells were transferred into a 24-well plate; till the hybridoma cells therein grew well, they could be cryopreserved.

### S6, production of monoclonal antibody:

The established hybridoma cells were seeded in 10% fetal bovine serum (FBS) supplemented DMEM at a density of 10e6/ml for scale-up culture; cells were centrifuged every 48 h and seeded in a fresh medium at a density of 10e6/ml again after supernatant was discarded. After the acquisition of a sufficient amount of cells, the original medium was removed by centrifugation; then, the cells were seeded in serum-free DMEM at a density of 10e6/ml, cultured for 72 h, and centrifuged, and the supernatant was collected.

### S7, purification of monoclonal antibody:

The cell culture supernatant where cells were removed by centrifugation was purified by means of HiTrap protein G affinity chromatography, and therapeutic mouse anti-human ErbB2 monoclonal antibody 10A6 was acquired.

### Embodiment 2

### Titration of mouse anti-human ErbB2 monoclonal antibody 10A6

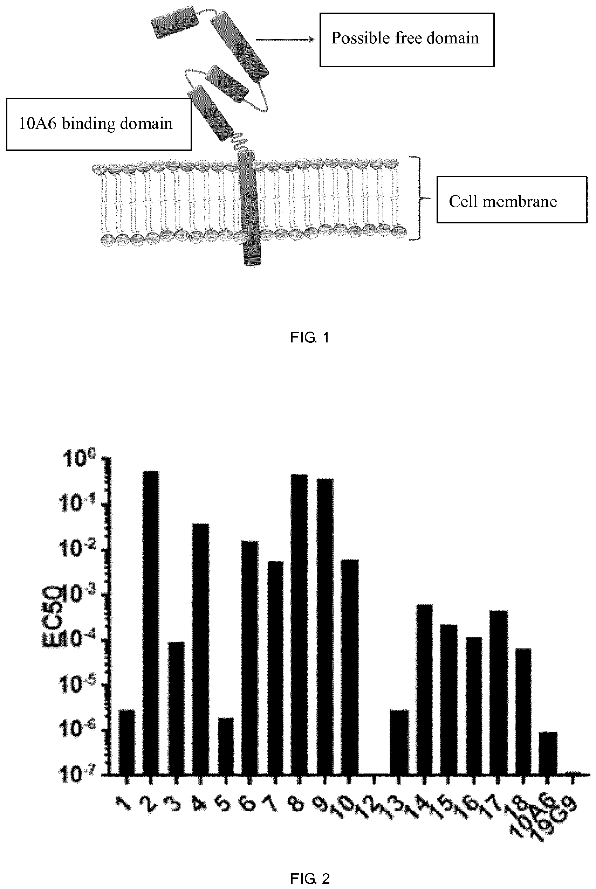

Gradient dilution was conducted using the acquired therapeutic mouse anti-human ErbB2 monoclonal antibody 10A6, and titration was conducted by means of indirect ELISA. The method for titrating the monoclonal antibody by means of indirect ELISA was to: coat the ErbB2-Domain4 protein at a density of 100 ng/well, dilute the mouse anti-human ErbB2 monoclonal antibody 10A6 at 1:10000, 1:20000, 1:40000, 1:80000, 1:160000, 1:320000, 1:640000, 1:1280000, 1:2560000, 1:5120000, 1:10240000, and 1:20480000, respectively, and measure their absorbance at A450 nm by means of indirect ELISA. The titer was the maximum dilution of the mouse anti-human ErbB2 monoclonal antibody 10A6 reacting with target antigen of immune protein, and the ratio of well reading to negative control value being >2.1 was determined as positive. EC50 values were calculated. Results are shown in FIG. 2. The EC50 value of the mouse anti-human ErbB2 monoclonal antibody 10A6 is 100 nM.

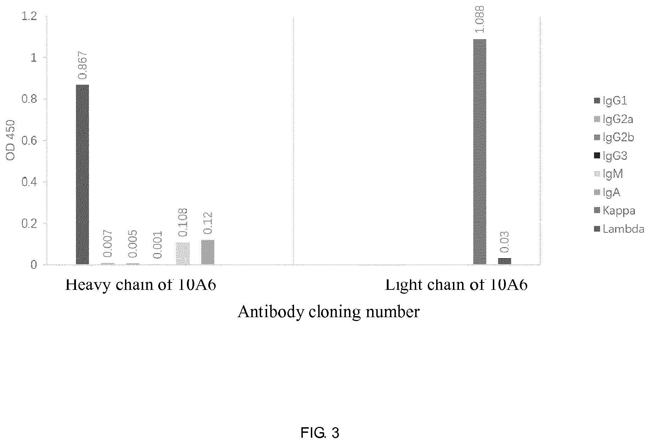

For the acquired mouse anti-human ErbB2 monoclonal antibody 10A6, Ig subclasses of monoclonal antibodies of anti-human ErbB2 protein were identified using Ig class and subclass ELISA kits. Results are shown in FIG. 3. Mouse IgG1-Kappa antibody is determined.

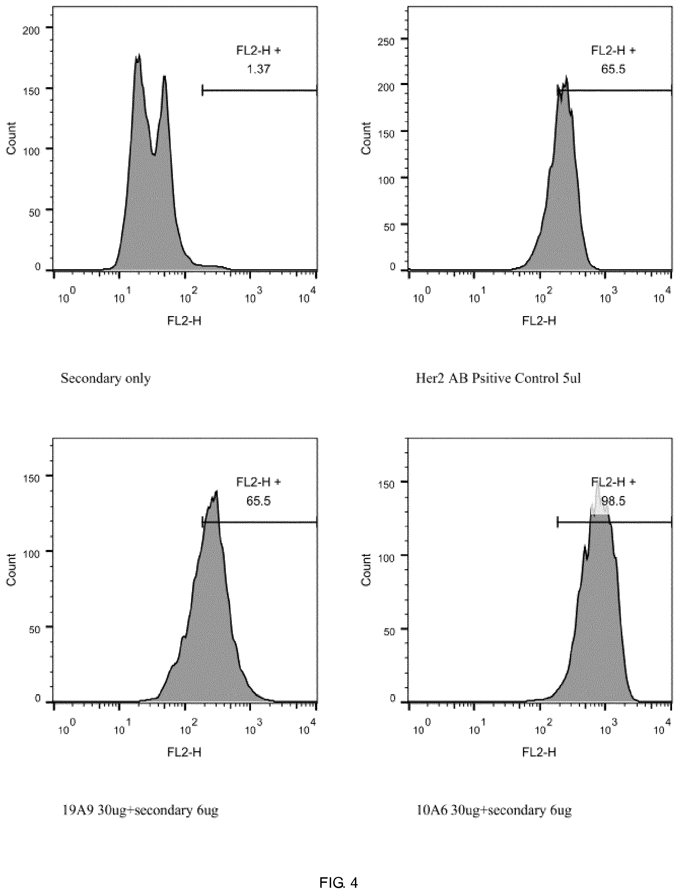

The affinity of hybridoma antibody for the target cell was analyzed by means of flow cytometry. After co-incubation of 30 µg each of clonal antibody proteins with 10⁶ ErbB2-positive target cells SKBR3 cells (purchased from Cell Resource Center, Institute of Basic Medical Sciences of the Chinese Academy of Medical Sciences) for 30 min, 6 µg of PE-conjugated anti-mouse secondary antibody (PE Goat anti-mouse IgG, Biolegend) was added to incubate for another 15 min, and measurements were made by means of flow cytometry. Results are shown in FIG. 4 and indicate that the mouse anti-human ErbB2 monoclonal antibody 10A6 has a high affinity for naturally conformational ErbB2 protein. In the figure, the left upper panel shows a negative control adding secondary antibody alone; the right upper panel shows that cells are successfully labeled after adding 5 µL of commercially PE-conjugated HER2 antibody for flow cytometry; the left lower panel shows that 19G9 clonal antibody protein can successfully label target cells; the right lower panel shows that 10A6 clonal antibody protein can successfully label target cells, and has a higher positive rate than other groups.

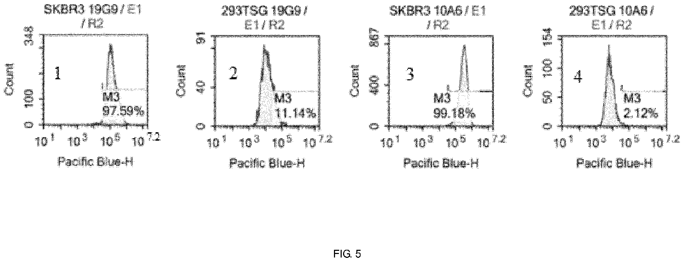

Specific binding of hybridoma antibody to target cell was analyzed by means of flow cytometry. After co-incubation of 1 µg each of clonal antibody proteins with 100000 ErbB2-positive (SKBR3 cells) and ErbB2-negative target cells (293T cells, purchased from Cell Resource Center, Institute of Basic Medical Sciences of the Chinese Academy of Medical Sciences) for 30 min, respectively, 0.1 µg of Pacific Blue conjugated anti-mouse secondary antibody was added to incubate for another 15 min, and measurements were made by means of flow cytometry. Results are shown in FIG. 5, and indicate that the mouse anti-human ErbB2 monoclonal antibody 10A6 has a high affinity for ErbB2-positive target cells and weakly binds to ErbB2-negative target cells, with good specificity. It can be seen from FIGS. 5-1 to 5-3 that both mouse anti-human ErbB2 monoclonal antibodies 10A6 and 10G9 have a high affinity for ErbB2-positive target cells, but the 10A6 depicted in FIG. 5-4 non-specifically binds to 293 cells, far below the non-specific binding of 19G9 depicted in FIG. 5-2.

The foregoing descriptions are only preferred implementation manners of the present invention. It should be noted that for a person of ordinary skill in the art, several improvements and modifications may further be made without departing from the principle of the present invention. These improvements and modifications should also be deemed as falling within the protection scope of the present invention.

## Claims

1. A mouse anti-human ErbB2 monoclonal antibody 10A6, wherein a heavy-chain amino acid sequence of the mouse anti-human ErbB2 monoclonal antibody 10A6 is depicted in SEQ ID NO.1, and a light chain amino acid sequence thereof is depicted in SEQ ID NO.2.

2. A gene encoding the mouse anti-human ErbB2 monoclonal antibody 10A6 according to claim 1, wherein a nucleotide sequence encoding the heavy chain of the mouse anti-human ErbB2 monoclonal antibody 10A6 is depicted in SEQ ID NO.3, and a nucleotide sequence encoding the light chain thereof is depicted in SEQ ID NO.4.

3. A preparation method of the mouse anti-human ErbB2 monoclonal antibody 10A6 according to claim 1, comprising the following steps of:
1) immunizing mice with ErbB2-Domain4-encoded protein as an antigen to acquire immune spleen cells;
2) fusing the immune spleen cells with myeloma cells to acquire hybridoma cells; and
3) culturing the hybridoma cells, collecting cell culture supernatant, and purifying to acquire the mouse anti-human ErbB2 monoclonal antibody 10A6.

4. The preparation method according to claim 3, wherein a nucleotide sequence of the ErbB2-Domain4 gene is depicted in SEQ ID NO.5.

5. The preparation method according to claim 3 or 4, wherein an amino acid sequence of the ErbB2-Domain4-encoded protein is depicted in SEQ ID NO.6.

6. An application of the mouse anti-human ErbB2 monoclonal antibody 10A6 according to claim 1 in the preparation of drugs for diagnosing or treating ErbB2-overexpressed malignancies.

7. An application of the gene encoding the mouse anti-human ErbB2 monoclonal antibody 10A6 according to claim 2 in the preparation of drugs for diagnosing or treating ErbB2-overexpressed malignancies.
